# EUROPÄISCHE PATENTSCHRIFT

(11) **EP 0 344 561 B1**
(45) Veröffentlichungstag und Bekanntmachung des Hinweises auf die Patenterteilung: **04.08.1993**
(21) Anmeldenummer: 89109144.9
(22) Anmeldetag: 20.05.1989
(51) Int. Cl.: C07C 31/20, C07C 29/00, C07C 31/24

(54) **Verfahren zur Herstellung niederer mehrwertiger Alkohole**
Process for the preparation of lower polyvalent alcohols
Procédé de préparation d'alcools polyvalents inférieurs

(30) Priorität: 28.05.1988 DE 3818198
(43) Veröffentlichungstag der Anmeldung: 06.12.1989
(73) Patentinhaber: BASF Aktiengesellschaft, 67063 Ludwigshafen (DE)
(72) Erfinder: Schuster, Ludwig, Dr., D-6703 Limburgerhof (DE); Himmele, Walter, Dr., D-6909 Walldorf (DE)

(56) Entgegenhaltungen:
- EP-A- 0 224 872
- DE-A- 2 321 101
- FR-A- 2 603 276

## Beschreibung

Die vorliegende Erfindung betrifft ein neues Verfahren zur Herstellung niederer mehrwertiger Alkohole durch katalytische Hydrogenolyse von Saccharose in wäßrigem Medium.

Mehrwertige Alkohole wie Ethylenglycol und 1,2-Propylenglycol, sind wesentliche Bestandteile in Frostschutzmitteln und wertvolle Ausgangskomponenten für technisch wichtige Veresterungs- und Veretherungsprodukte, von denen wegen seiner Bedeutung als Faserrohstoff der Polyester aus Ethylenglycol und Terephthalsäure besonders hervorzuheben ist.

Es ist bekannt (G.v.Ling et al, Ind. Eng. Chem. Prod. Res. Develop. 9, 2 [1970] S. 211) Saccharose, gelöst in einem Methanol/Wasser-Gemisch, mit einem CuO-CaO₂-SiO₂-Katalysator hydrierend zu spalten, wobei im organischen Reaktionsaustrag rd. 31 Gew.% Glycerin, 16 Gew.% Ethylenglycol, 18 Gew.% 1,2-Propylenglycol, 16 Gew.% Hexite und 19 Gew.% andere Produkte erhalten werden.

Verwendet man als Lösungsmittel Ethanol und als Katalysator Kupferchromit, entstehen nach G. Natta et al in Chem. Berichte 76, 7 [1943] Tafel 1, S. 644, bezogen auf die eingesetzte Menge Saccharose, neben Sorbit und nicht destillierbaren mehrwertigen Alkoholen bis zu 54 Gew.% 1,2-Propylenglycol und 14 Gew.% Glycerin.

Nach R. Weidenhagen und H. Wegner, Chem. Berichte 71, 12 [1938], S. 2712 ff. läßt sich die Bildung von Glycerin zugunsten von 1,2-Propylenglycol gänzlich unterdrücken, wenn man die Hydrogenolyse in zwei Stufen durchführt. In der ersten Stufe wird die wäßrige Saccharoselösung in Gegenwart eines mit Molybdän aktivierten Nickel-Katalysators in neutralem Milieu hydriert. Dann unterbricht man die Reaktion und destilliert das gebildete Hydroxyaceton ab. Dieses wird anschließend in wäßriger Lösung in Gegenwart des gleichen Katalysators nach Zusatz von Calciumhydroxid zu Ende hydriert. Aus 68,4 g Saccharose wurden auf diese Weise 25 g Propylenglycol gewonnen.

Der vorliegenden Erfindung lag nun die Aufgabe zugrunde, ein einstufiges Verfahren zur katalytischen Hydrogenolyse von Saccharose in wäßriger Lösung zur Verfügung zu stellen, bei dem man Ethylenglycol und 1,2-Propylenglycol als Hauptbestandteile erhält.

Demgemäß wurde ein Verfahren zur katalytischen Hydrogenolyse von Saccharose in wäßriger Lösung gefunden, welches dadurch gekennzeichnet ist, daß man einen Katalysator verwendet, dessen aktive Masse, berechnet auf den Gehalt der Metalle, aus
0 bis 100 Gew.% Cobalt
0 bis 85 Gew.% Kupfer und
0 bis 80 Gew.% Mangan
besteht.

Definitionsgemäß können die erfindungsgemäß zu verwendenden Katalysatoren Cobalt als einziges Metall enthalten oder nur aus zwei Metallen bestehen, jedoch hat es sich als vorteilhaft erwiesen, wenn die Katalysatoren alle drei Metalle enthalten, wobei der Mangananteil mindestens 5 Gew.% beträgt.

Gruppen von Katalysatoren mit besonders guter Eignung entsprechen den folgenden Zusammensetzungen:
a) 5 bis 90 Gew.% Co
   5 bis 80 Gew.% Cu
   5 bis 75 Gew.% Mn
b) 0 bis 5 Gew.% Co
   25 bis 75 Gew.% Cu
   20 bis 75 Gew.% Mn

Bevorzugt werden für das erfindungsgemäße Verfahren Katalysatoren eingesetzt, die dadurch erhältlich sind, daß man die betreffenden Metalle in Form von wäßrigen Salzlösungen mischt, sie aus dieser Mischung durch Zugabe einer Base gemeinsam fällt, die ausgefällte Masse abtrennt, trocknet und durch Calzinieren in ein Mischoxid umwandelt und gewünschtenfalls durch Reduktion mit Wasserstoff aktiviert.

Zur Herstellung der wäßrigen Ausgangslösung der betreffenden Metalle werden zweckmäßigerweise deren Nitrate, Sulfate oder Acetate verwendet. Die Fällung aus der wäßrigen Salzlösung erfolgt bevorzugt durch Zugabe wasserlöslicher Hydroxide oder Carbonate, wobei Alkalimetallcarbonate, insbesondere Na₂CO₃, besonders bevorzugt sind.

Mit besonderem Vorteil wird die Fällung gemäß der DE-C2 23 21 101 auf die Weise ausgeführt, daß man in einer ersten Stufe die wäßrige Ausgangslösung der betreffenden Metalle mittels einer Alkalimetallcarbonatlösung bei einer Temperatur zwischen 40 und 70°C auf einen pH-Wert von wenigstens 8 bringt und danach in einer zweiten Stufe die Fällung durch vorsichtiges Ansäuern, d.h. Zugabe von zusätzlicher wäßriger Metallsalzlösung, vervollständigt, wobei ein pH-Wert von 6,8 bis 7,5 eingehalten werden sollte.

Die gefällte Masse wird gewöhnlich durch Filtration gewonnen, gewaschen und getrocknet. Anschließend wird zweckmäßigerweise bei 420 bis 550°C mit Luft calziniert.

Zur Aktivierung des bei der Calzinierung erhaltenen Katalysators wird dieser in allgemein bekannter Weise bei erhöhter Temperatur, vorteilhaft zwischen 200 und 600°C, im Wasserstoffstrom reduziert. Diese Vorgehensweise ist gegenüber einer Aktivierung in situ, d.h. einer allmählichen Aktivierung im Verlauf der Hydrogenolyse, bevorzugt.

Vorzugsweise werden die Katalysatoren als Vollkatalysatoren eingesetzt, d.h. der gesamte Katalysator besteht aus der katalytisch aktiven Masse. Die katalytisch aktive Masse kann aber auch auf inerte Träger aufgebracht sein. Man stellt solche Trägerkatalysatoren beispielsweise her, indem man die aktiven Bestandteile aus ihrer gemeinsamen wäßrigen Lösung in Form von Hydroxiden oder Carbonaten auf dem Träger niederschlägt, trocknet und wie beschrieben calziniert und aktiviert.

Das erfindungsgemäße Verfahren ist diskontinuierlich oder kontinuierlich durchführbar. Bei der kontinuierlichen Verfahrensweise leitet man die wäßrige Saccharoselösung und den Wasserstoff zweckmäßigerweise über einen auf Reaktionstemperatur erhitzten Festbettkatalysator. Die Katalysatorschüttung kann aus verschiedenen Formkörpern bestehen, die beispielsweise dadurch erhältlich sind, daß man das nach der Calcinierung erhaltene Mischoxid pulverisiert und das erhaltene Oxidpulver beispielsweise mit einer wäßrigen Lösung einer anorganischen Säure, die Polysäuren zu bilden vermag, zu einer Paste verarbeitet, diese zu Strängen, Perlen oder Tabletten verformt und danach in üblicher Weise calziniert und reduziert.

Für die diskontinuierliche Verfahrensweise, bei der man beispielsweise die wäßrige Saccharoselösung und den Katalysator in ein Druckgefäß einbringt und unter Wasserstoffatmosphäre auf die Reaktionstemperatur erhitzt, wird der aktivierte Katalysator zweckmäßigerweise in Pulverform eingesetzt.

Zur Herstellung des Pulvers wird gewöhnlich die Naßvermahlung angewendet, da die aktivierten Katalysatoren pyrophor sind. Bevorzugt wird eine mittlere Korngröße von 0,005 bis 0,5 mm (größter Durchmesser der Teilchen). Überlicherweise werden pro kg einer 50 gew.%igen wäßrigen Saccharoselösung 30 bis 100, bevorzugt 50 bis 70 g aktivierter Pulverkatalysator eingesetzt.

Um Katalysatoren mit vorteilhaften mechanischen Eigenschaften zu erhalten, werden der wäßrigen Ausgangslösung zweckmäßigerweise anorganische Säuren, die Polysäuren zu bilden vermögen, beispielsweise Schwefelsäure, Borsäure, Phosphorsäure, Molybdänsäure, Wolframsäure und/oder deren Salze wie Trinatriumphosphat, Natriumtetraborat, Kaliumdihydrogenphosphat, Calziumhydrogenphosphat, Magnesiumhydrogenborat, Aluminiumphosphat, Natriummolybdat, Ammoniummolybdat, Ammoniumvanadat und Natriumwolframat zugesetzt.

Eine andere Möglichkeit der Zugabe dieser Zusatzstoffe besteht darin, die calzinierte Masse mit einer wäßrigen Lösung des entsprechenden Salzes der anorganischen Säure zu tränken und anschließend mit einer Mineralsäure, z.B. Salpetersäure, zu behandeln. Danach wird getrocknet, wie beschrieben im Wasserstoffstrom reduziert und naß vermahlen. Der Anteil der anorganischen Säure und/oder deren Salze beträgt im allgemeinen 0,1 bis 15 Gew.%, bezogen auf die reduzierte Form des Katalysators. Derartige Zusatzstoffe enthaltende Katalysatoren haben im Anwendungsfalle als Katalysatorpulver bei intensivem Rühren beispielsweise eine geringere Neigung zum Verklumpen.

Um die wirksame Oberfläche und damit die Aktivität der katalytisch aktiven Komponenten zu erhöhen, können als weitere Zusatzstoffe vor der Calzinierung schwer reduzierbare Oxide wie MgO, CaO oder Al₂O₃ zugegeben werden, deren Gewichtsanteil bis zu 50 Gew.% betragen kann, bezogen auf die aktivierte Form des Kontaktes. Durch diese Aktivitätserhöhung kann aber die Selektivität der hydrierenden Spaltung bezüglich der gewünschten niederen Alkohole vermindert werden, so daß man vorzugsweise ohne diese Zusatzstoffe oder nur mit geringen Mengen davon - etwa bis zu 10 Gew.% - arbeitet.

Es wurde weiterhin gefunden, daß die Selektivität von der Reaktionstemperatur abhängt. Besonders hohe Ausbeuten an Ethylenglycol und 1,2-Propylenglycol erhält man zwischen 220 und 280°C, insbesondere zwischen 240 und 270°C.

Bei höheren Temperaturen erfolgt zunehmend weitergehende Hydrogenolyse zu einwertigen Alkoholen wie Ethanol, Isopropanol, Butan-2-ol oder verschiedenen Hexanolen. Teilweise entstehen dann auch Kohlenwasserstoffe wie Methan. Bei tieferen Temperaturen findet man neben Sorbit, Mannit und Hexan-1,2,6-triol insbesondere Hexan-1,2,5,6-tetrol in erhöhten Mengen als Nebenprodukte.

Hexan-1,2,5,6-tetrol ist eine wertvolle Ausgangsverbindung für die Polyurethanchemie. Sein Anteil kann bei Reaktionstemperaturen zwischen 200 und 240°C, insbesondere zwischen 220 und 230°C, bezogen auf die Gesamtheit der organischen Bestandteile im Reaktionsaustrag, bis zu 25 Gew.% betragen.

Vom Wasserstoffdruck hängt die Selektivität des erfindungsgemäßen Verfahrens wie folgt ab. Bei Drücken unterhalb von 250 bar fällt die Selektivität unter zunehmender Bildung von Hexiten deutlich ab und oberhalb von 700 bar nimmt die Tendenz zur weitergehenden Hydrolyse unter Bildung von einwertigen Alkoholen und Kohlenwasserstoffen zu. Insbesondere im Bereich zwischen 300 und 700 bar ist die Selektivität jedoch nahezu druckunabhängig. Anwendungstechnisch bevorzugt ist der Bereich zwischen 250 und 350 bar.

Die Konzentration der wäßrigen Saccharose-Lösung hat auf die Produktverteilung nur einen geringen Einfluß. Erst bei Konzentrationen oberhalb von 70 Gew.% Saccharose nimmt die Selektivität des erfindungsgemäßen Verfahrens deutlich ab. Bevorzugt werden Konzentrationen zwischen 30 und 60 Gew.%.

Weiterhin ist es zweckmäßig, für eine intensive Durchmischung des Reaktionsgutes zu sorgen, wie sie beispielsweise durch Verwendung eines schnellaufenden Turbinenrührers beim Arbeiten im Druckgefäß erzielt werden kann. Der Gang der Umsetzung kann anhand des Wasserstoffdruckes verfolgt werden. Wenn nur noch wenig Wasserstoff aufgenommen wird, ist die Umsetzung beendet. Dieser Zeitpunkt wird gewöhnlich nach wenigen Stunden erreicht.

Unter den angegebenen Vorzugsbedingungen erhält man in der Regel, bezogen auf die Gesamtheit der organischen Bestandteile im Reaktionsaustrag, folgende Ausbeuten:

| | |
|---|---|
| 1,2-Propylenglycol | 50 bis 65 Gew.% |
| Ethylenglycol | 20 bis 25 Gew.% |
| 1,2-Butylenglycol | 5 bis 7 Gew.% und |
| Hexan-1,2,5,6-tetrol | 3 bis 10 Gew.% |
| Glycerin | < 1 Gew.% |

Die Umsetzungen erfolgten vollständig, d.h. Saccharose konnte nicht mehr nachgewiesen werden.

### Beispiel

### a) Herstellung des Katalysators

Der pH-Wert einer sauren Ausgangslösung von 23,78 kg Kobalt(II)nitrat-Hexahydrat, 4,65 kg Kupfer(II)nitrat-Hexahydrat, 1,83 kg Mangan(II)nitrat-Hexahydrat und 0,20 kg einer 75,3 gew.%igen wäßrigen Phosphorsäure in 17,5 kg Wasser wurde unter Rühren durch Zugabe einer 20 gew.%igen wäßrigen Natriumcarbonatlösung zunächst auf 8,5 erhöht und anschließend durch Zugabe zusätzlicher Ausgangslösung auf 7 reduziert. Der dabei angefallende Niederschlag wurde abfiltriert, gewaschen, getrocknet und bei einer Temperatur von 500°C mit Luft calziniert. Anschließend wurden 4 kg der calzinierten Masse pulverisiert und mit 626 g einer Löusng von 166 g Molybdäntrioxid in 460 g 20 gew.%igem Ammoniak sowie mit 1,58 kg einer 11,7 gew.%igen Salpetersäure vermischt.

Die erhaltene Masse wurde zu Strängen verformt, getrocknet, in üblicher Weise calziniert, reduziert und danach naß zu einem Pulver einer mittleren Korngröße von 0,1 mm vermahlen. In nicht reduzierter Form wies der Katalysator folgende Zusammensetzung auf:

| | |
|---|---|
| 66,8 Gew.% CoO | (= 72 Gew.% Co)* |
| 19,2 Gew.% CuO | (= 21 Gew.% Cu)* |
| 7,1 Gew.% Mn₃O₄ | (= 7 Gew.% Mn)* |
| 3,4 Gew.% H₃PO₄ und | |
| 3,5 Gew.% MoO₃ | |

| | |
|---|---|
| *bezogen auf Co+Cu+Mn = 100 %. | |

### b) Durchführung der katalytischen Hydrogenolyse

In einem Druckgefäß von 250 ml Inhalt wurden 11,5 g des feinteiligen, aktivierten Katalysators a) in 140 g einer 50 gew.%igen wäßrigen Saccharoselösung suspendiert.

Anschließend wurde mit Wasserstoff ein Druck von 180 bar eingestellt und unter intensivem Rühren innerhalb von 15 Minuten auf die Reaktionstemperatur von 250°C aufgeheizt. Beim Erreichen von 180°C zeigte intensive Gasaufnahme das Einsetzen der Reaktion an. Der mit dieser Gasaufnahme einhergehende Wasserstoffdruckabfall wurde unmittelbar ausgeglichen und im weiteren Verlauf der Reaktion auf entsprechende Art und Weise zwischen 280 und 300 bar gehalten.

Nach 30 min verlangsamte sich die Reaktion und nach einer Gesamtreaktionsdauer von 4,5 h wurde die Umsetzung durch Abkühlen und Entspannen des Druckgefäßes abgebrochen. Der Katalysator wurde vom Reaktionsaustrag abfiltriert und mittels HPLC und GC analysiert. Zusätzlich wurde nach Karl Fischer der Wassergehalt bestimmt.

Die Analyse ergab 59 Gew.% H₂O und 41 Gew.% organische Bestandteile.

Als organische Bestandteile wurden ermittelt:
60 Gew.% 1,2-Propylenglycol
20 Gew.% Ethylenglycol
7 Gew.% 1,2-Butylenglycol
5 Gew.% Hexan-1,2,5,6-tetrol
1 Gew.% Hexan-1,2,6-triol
4 Gew.% einwertige Alkohole wie Ethanol, n-Propanol und 2-Butanol
3 Gew.% nicht identifizierte Verbindungen.

## Patentansprüche

1. Verfahren zur Herstellung niederer mehrwertiger Alkohole durch katalytische Hydrogenolyse von Saccharose in wäßrigem Medium, dadurch gekennzeichnet, daß man einen Katalysator verwendet, dessen aktive Masse, berechnet auf den Gehalt der Metalle, aus
0 bis 100 Gew.% Cobalt
0 bis 85 Gew.% Kupfer und
0 bis 80 Gew.% Mangan
besteht.

2. Verfahren nach Anspruch 1, dadurch gekennzeichnet, daß man einen Katalysator verwendet, der dadurch erhältlich ist, daß man die betreffenden Metalle in Form von Salzlösungen mischt, sie aus dieser Mischung durch Zugabe einer Base gemeinsam fällt, die ausgefällte Masse abtrennt, trocknet und durch Calzinieren in ein Mischoxid umwandelt und gewünschtenfalls durch Reduktion mit Wasserstoff aktiviert.

3. Verfahren nach Anspruch 1 oder 2, dadurch gekennzeichnet, daß die aktive Masse des verwendeten Katalysators mindestens 5 Gew.% Mangan enthält.

4. Verfahren nach Anspruch 2, dadurch gekennzeichnet, daß der verwendete Katalysator als Zusatzstoff eine anorganische Polysäure enthält.

5. Verfahren nach Anspruch 2, dadurch gekennzeichnet, daß der verwendete Katalysator als Zusatzstoffe schwer reduzierbare Oxide enthält.

6. Verfahren nach den Ansprüchen 1 bis 5, dadurch gekennzeichnet, daß man das Verfahren bei einer Temperatur zwischen 220 und 280°C durchführt.

7. Verfahren nach den Ansprüchen 1 bis 6, dadurch gekennzeichnet, daß man das Verfahren bei einem Wasserstoffdruck zwischen 250 und 700 bar durchführt.

## Claims

1. A process for the preparation of a lower polyhydric alcohol by catalytic hydrogenolysis of sucrose in an aqueous medium, wherein a catalyst is used whose active material consists of
from 0 to 100% by weight of cobalt,
from 0 to 85% by weight of copper and
from 0 to 80% by weight of manganese,
the percentages being based on the content of the metals.

2. A process as claimed in claim 1, wherein the catalyst used is obtainable by a procedure in which the relevant metals are mixed in the form of salt solutions and are precipitated together from this mixture by adding a base, the precipitated material is isolated, dried and converted by calcination into a mixed oxide and, if desired, the latter is activated by reduction with hydrogen.

3. A process as claimed in claim 1, wherein the active material of the catalyst used contains not less than 5% by weight of manganese.

4. A process as claimed in claim 2, wherein the catalyst used contains an inorganic polyacid as an additive.

5. A process as claimed in claim 2, wherein the catalyst used contains poorly reducible oxides as additives.

6. A process as claimed in claim 1, which is carried out at from 220 to 280°C.

7. A process as claimed in claim 1, which is carried out under a hydrogen pressure of from 250 to 700 bar.

## Revendications

1. Procédé de préparation d'alcools polyhydroxylés inférieurs par l'hydrogénolyse catalytique du saccharose en milieu aqueux, caractérisé en ce que l'on utilise un catalyseur dont la masse active, calculée sur la teneur en métaux, se compose de
O à 100% en poids de cobalt,
0 à 85% en poids de cuivre et
0 à 80% en poids de manganèse.

2. Procédé suivant la revendication 1, caractérisé en ce que l'on utilise un catalyseur que l'on peut obtenir par le fait que l'on mélange les métaux concernés sous forme de solutions de sels, on les sépare de ce mélange par précipitation en commun par l'addition d'une base, on sépare la masse précipitée, on la sèche et on la convertit en oxyde mixte par calcination et on active éventuellement l'oxyde mixte par réduction avec l'hydrogène.

3. Procédé suivant la revendication 1 ou 2, caractérisé en ce que la masse active du catalyseur utilisé contient au moins 5% en poids de manganèse.

4. Procédé suivant la revendication 2, caractérisé en ce que le catalyseur utilisé contient un polyacide inorganique à titre d'additif.

5. Procédé suivant la revendication 2, caractérisé en ce que le catalyseur utilisé contient des oxydes difficilement réductibles à titre d'additifs.

6. Procédé suivant les revendications 1 à 5, caractérisé en ce qu'on entreprend le procédé à une température qui fluctue de 220 à 280°C.

7. Procédé suivant les revendications 1 à 6, caractérisé en ce que l'on entreprend le procédé sous une pression d'hydrogène qui varie de 250 à 700 bars.
